# EUROPEAN PATENT APPLICATION

(11) **EP 2 184 144 A1**
(43) Date of publication of application: **12.05.2010**
(21) Application number: 08168486.2
(22) Date of filing: 06.11.2008
(51) Int. Cl.: B27N 3/00, C07C 263/20, C08G 18/64, C08G 18/76, C08L 97/02

(54) **Polyisocyanate composition used for binding lignocellulosic materials**

(71) Applicant: HUNTSMAN INTERNATIONAL LLC, Salt Lake City, Utah 84108 (US)
(72) Inventor: The designation of the inventor has not yet been filed
(74) Representative: Swinnen, Anne-Marie

(57) **Abstract**

Polyisocyanate composition for binding lignocellulosic materials comprising a methylene bridged polyphenyl polyisocyanate composition having a having a relative content of minor triisocyanate isomers of at least 17 wt% based on the total triisocyanate content.

## Description

This invention relates to polyisocyanate compositions and, in particular, to polyisocyanate compositions for use in binding lignocellulosic material used in the manufacture of wafer board (known extensively as oriented strand board), medium density fiberboard and particle board (also known as chipboard).

The use of organic polyisocyanates as binders for lignocellulosic material in the manufacture of sheets or molded bodies such as wafer board, chipboard, fiberboard and plywood is well known and is commercially desirable because the resulting composites have high adhesive and cohesive strength, flexibility to changes in wood species, versatility with respect to cure temperature and rate, excellent structural properties of the resulting composites and the ability to bond with lignocellulosic materials having higher water content than typically used for condensation resins such as phenol formaldehyde. In a typical process the organic polyisocyanate, optionally in the form of a solution, dispersion or aqueous emulsion, is applied to the lignocellulosic material which is then subjected to heat and pressure.

Preferred isocyanates are aromatic diisocyanates or polyisocyanates of higher functionality such as pure diphenylmethane diisocyanate or mixtures of methylene bridged polyphenyl polyisocyanates containing diisocyanates, triisocyanates and higher functionality polyisocyanates. Methylene bridged polyphenyl polyisocyanates are well known in the art. They are prepared by phosgenation of corresponding mixtures of polyamines obtained by condensation of aniline and formaldehyde. For convenience, polymeric mixtures of methylene bridged polyphenyl polyisocyanates containing diisocyanate, triisocyanate and higher functionality polyisocyanates are referred to hereinafter as polymeric MDI.

The composition of polymeric MDI can be partly described in terms of the relative amounts of the various isomers of the various molecular weight homologues.
Thus, for example, for the diisocyanates, reference can be made to the relative amounts of the 4,4'-MDI, 2,4'-MDI and 2,2'-MDI isomers. The 2,4'-MDI and 2,2'-MDI isomers can be considered together, their abundances can be summed and they can thence be referred to as the minor isomers and compared to the content of the major 4,4'-MDI.

Likewise, for the triisocyanates present in polymeric MDI, a specific major isomer is invariably present in polymeric MDI's manufactured commercially which is produced by phosgenation of the major triamine isomer formed in the acid catalysed polyamine production process as described in Chemistry and Technology of Isocyanates, H. Ulrich, John Wiley & Sons, 1996 [ISBN 0-471-96371-2]; the other isomers [see Ulrich] may be considered in total together as the minor triisocyanate isomers. The ratios of the various isomers of the still higher molecular weight oligomers (tetra-, penta-, hexa-isocyanates, etc.) also vary in ways related to the manufacturing processes but the number of isomers of each homologue present in the polymeric MDI make their determination more problematic. Determination of the separate quantities of diisocyanate isomers and triisocyanate isomers can be carried out by means of gas chromatography as is practised widely in the polyurethanes industry and well known to those skilled in the art. Thus this aspect of the characterisation of polymeric MDI is conveniently based on the diisocyanate and triisocyanate isomer abundances. It is to be understood that the further description of polymeric MDI compositions in these terms includes by inference the associated changes in the isomer ratios of all the higher molecular weight species normally present.

Commercially available polymeric MDI's containing relatively high levels of minor triisocyanate isomers are known. However, standard polymeric MDI normally has a relative minor triisocyanate isomer content of less than 17 wt% (based on the total triisocyanate content).

The polyisocyanate binder compositions should be able to provide for composites with superior dimensional stability when in contact with moisture.

However the dimensional stability achieved (thickness swelling in the presence of moisture and shrinkage) is not quite sufficient when standard polymeric MDI compositions are used.

Therefore it is an object of the present invention to provide polyisocyanate compositions for binding lignocellulosic materials that maximise thickness swell performance without adversely affecting other performance characteristics such as bonding strength.

The present invention provides a polyisocyanate composition for binding lignocellulosic materials comprising methylene bridged polyphenyl polyisocyanates having a relative content of minor triisocyanate isomers of at least 17 wt% , preferably at least 20 wt% and most preferably at least 22 wt% based on the total triisocyanate content.

In terms of absolute content of minor triisocyanate isomers these numbers generally correspond to at least 4 wt%, preferably at least 4.5 wt%, and most preferably at least 4.8 wt% based on the total methylene bridged polyphenyl polyisocyanates.

By using polymeric MDI with such a high content of minor triisocyanate isomers a substantial reduction (5 to 18 %) in thickness swell of the lignocellulosic bodies bound with said polymeric MDI is achieved at the same overall resin loading.
Alternatively a substantial reduction (at least 25 %) in resin loading is possible while maintaining the swelling behaviour at the same performance level, hence leading to an economic benefit.
And at the same time all the other properties remain similar or at least are not detrimentally affected.

The polyisocyanate composition of the present invention containing such high content of minor triisocyanate isomers species can be obtained by phosgenation of a suitable polyaromatic polyamine (DADPM) manufactured using suitable ratios of aniline, formaldehyde and acidic catalyst (frequently HCl) and carried out under appropriate reaction process conditions and, optionally, with removal of some fraction of the diisocyanates from the polymeric polyisocyanate mixture resulting from phosgenation of the DADPM. Particularly suitable for the manufacture of DADPM with high levels of the minor isomers are processes generally employing relatively high temperatures, often with correspondingly lower levels of catalyst, as is well known in the art as taught in, for example, GB 1167984 and DE 3407494 and described generally in Chemistry and Technology of Isocyanates, H. Ulrich.

The polymeric MDI composition of the present invention preferably has a difunctional MDI isomer content of between 35 and 55 wt%, more preferably 38 to 52 wt%, most preferably 40 to 48 wt%.

The polymeric MDI composition of the present invention can also be a water-emulsifiable one as described, for example, in GB 1444933, EP 516361 and WO 91/03082. Especially for applications in medium density fiberboard the use of emulsifiable polyisocyanate compositions is preferred.

These emulsifiable polymeric MDI compositions of the present invention having a content of minor triisocyanate isomers in the presently claimed ranges can preferably be obtained by modifying a polymeric MDI containing minor triisocyanate isomers in the claimed ranges to become emulsifiable in any of the many possible ways known to those skilled in the art.

Modified polyisocyanates containing isocyanurate, carbodiimide or uretonimine groups may be employed as well. Further blocked polyisocyanates, like the reaction product of a phenol or an oxime and a polyisocyanate, may be used, having a deblocking temperature below the temperature applied when using the polyisocyanate composition.
The organic polyisocyanate may also be an isocyanate-ended prepolymer made by reacting an excess of a diisocyanate or higher functionality polyisocyanate with a polyol.

The polyisocyanate composition for use according to the present invention may be produced in accordance with any of the techniques known in the art. The minor triisocyanate isomer content of the polymeric MDI composition may be brought within the required ranges, if necessary, by techniques which are well known in the art.

The polyisocyanate binder composition may further contain any of the additives generally known in the art.
Conventional release agents such as polysiloxanes, saturated or unsaturated fatty acids or fatty acid amides or fatty acid esters or polyolefin wax can be added to the polyisocyanate composition of the present invention. By doing so the release performance from the press platens is improved; pre-treatment of the press platens with external release agents is another way to improve the release.
In order to further improve either the storage stability of the polyisocyanate composition or the cost effectiveness of the present invention a diluent may be added to the composition.

Preferred diluents are phthalates, aliphatic carboxylates, fatty acid esters, linseed oil, soybean oil and propylene carbonate.
The composition further may comprise conventional additives like flame retardants, lignocellulosic preserving agents, fungicides, bacteriocides, biocides, waxes, sizing agents, fillers, surfactants, thixotropic agents, curing aids, emulsifiers, wetting agents, coupling agents and other binders like formaldehyde condensate adhesive resins and lignin. The additives can be used in the amounts commonly known in the art.

The polyisocyanate composition of the present invention can be made by simply mixing the ingredients at room or elevated temperature or, when necessary, in case one of the ingredients is solid at room temperature, above the melting point of such an ingredient or by prior solubilisation in an apprpriate solvent unless otherwise required as a suspension.

The present invention is primarily concerned with a process for preparing lignocellulosic bodies by bringing lignocellulosic parts into contact with the present polyisocyanate composition and by pressing this combination.

The lignocellulosic bodies are prepared by bringing the lignocellulosic parts into contact with the polyisocyanate composition like by means of mixing, spraying and/or spreading the composition with/onto the lignocellulosic parts and by pressing the lignocellulosic parts, preferably by hot-pressing, normally at 120°C to 300°C, preferably 140°C to 270°C and 2 to 6 MPa specific pressure.
Such binding processes are commonly known in the art.

In wafer board manufacture the lignocellulosic material and the polyisocyanate composition may be conveniently mixed by spraying the present polyisocyanate composition on the lignocellulosic material while it is being agitated.
In medium density fibreboard the lignocellulosic material and the polyisocyanate composition may be conveniently mixed by spraying the present polyisocyanate composition on the lignocellulosic material in a blowline as commonly used.

The lignocellulosic material after treatment with the polyisocyanate composition is placed on caul plates made of aluminum or steel which serve to carry the furnish into the press where it is compressed to the desired extent usually at a temperature between 120°C and 300°C, preferably between 140°C and 270°C. At the start of a manufacturing run it may be helpful, but not essential, to condition the press platens by spraying their surfaces with an external release agent or to increase the cycle time of the first press load. A preconditioned press may then be used many times in the process of the invention without further treatment.

While the process is particularly suitable for the manufacture of wafer board known extensively as oriented strand board and will be largely used for such manufacture, the process may not be regarded as limited in this respect and can also be used in the manufacture of medium density fiberboard, particle board (also known as chipboard) and plywood.

Thus the lignocellulosic material used can include wood strands, woodchips, wood fibers, shavings, veneers, wood wool, cork, bark, sawdust and like waste products of the wood working industry as well as other materials having a lignocellulosic basis such as paper, bagasse, straw, flax, sisal, bamboo, coconut fibers, hemp, rushes, reeds, rice hulls, husks, grass, nutshells and the like. Additionally, there may be mixed with the lignocellulosic materials other particulate or fibrous materials such as grinded foam waste (for example, grinded polyurethane foam waste), mineral fillers, glass fiber, mica, rubber, textile waste such as plastic fibers and fabrics. These materials may be used in the form of granulates, shavings or chips, fibers, strands, spheres or powder.

When the polyisocyanate composition is applied to the lignocellulosic material, the weight ratio of polyisocyanate/lignocellulosic material will vary depending on the bulk density of the lignocellulosic material employed. Therefore, the polyisocyanate compositions may be applied in such amounts to give a weight ratio of polyisocyanate/lignocellulosic material in the range of 0.1:99.9 to 20:80 and preferably in the range of 0.5:99.5 to 10:90 and most preferably in the range 3:97 to 8:92.
By using the presently claimed polyisocyanate composition lower resin loadings (at least 25 % lower than standard loadings) can be used without dramatically deteriorating the thickness swell performance of the boards. Resin loadings between 1.5 and 6 wt% can thus be used.

If desired, other conventional binding agents, such as formaldehyde condensate adhesive resins, may be used in conjunction with the polyisocyanate composition.

More detailed descriptions of methods of manufacturing wafer board and medium density fibreboard and similar products based on lignocellulosic material are available in the prior art. The techniques and equipment conventionally used can be adapted for use with the polyisocyanate compositions of the present invention.

The sheets and molded bodies produced from the polyisocyanate compositions of the present invention have excellent mechanical properties and they may be used in any of the situations where such articles are customarily used.

The invention is illustrated but not limited by the following examples.
In these examples the following ingredients were used:
ISO 1: polymeric MDI modified with 3 % of monomethyl ether of polyethylene glycol of MW 750 rendering the product emulsifiable and having a difunctional MDI content of 44.2 wt%, an absolute content of minor triisocyanate isomers of 3.4 wt% (based on the total polyisocyanate) and a relative content of minor triisocyanate isomers of 14.8 wt% (based on the total triisocyanate content).
ISO 2: polymeric MDI modified with 3 % of monomethyl ether of polyethylene glycol of MW 750 rendering the product emulsifiable and having a difunctional MDI content of 43.5 wt%, an absolute content of minor triisocyanate isomers of 4.9 wt% (based on the total polyisocyanate) and a relative content of minor triisocyanate isomers of 22.8 wt% (based on the total triisocyanate content).
ISO 3: polymeric MDI having a difunctional MDI content of 40.3 wt%, an absolute content of minor triisocyanate isomers of 3.8 wt% (based on the total polyisocyanate) and a relative content of minor triisocyanate isomers of 16.8 wt% (based on the total triisocyanate content).
ISO 4: polymeric MDI having a difunctional MDI content of 46.1 wt%, an absolute content of minor triisocyanate isomers of 5.4 wt% (based on the total polyisocyanate) and a relative content of minor triisocyanate isomers of 23.8 wt% (based on the total triisocyanate content).

### EXAMPLE 1:

Emulsified compositions containing various polyisocyanates as identified below in Table 1 and water (50/50 wt/wt) were prepared.
These compositions were used to make medium density fibreboards using a dry blending method wherein the wood fibres are deballed, charged into the drum blender where upon resin is sprayed onto the wood whilst it is tumbling using an air assisted spray nozzle.
Commercially produced Eastern European mixed softwood fibres having a moisture content of 12% were used.
Resin loading of 4 wt% on total wood composite were used.
Wood panels with dimensions 40 x 40 x 1.2 cm were produced using a single step press to thickness press profile with press platens at 220°C; the total pressing time was 150 seconds. After producing the panels they were conditioned at 23°C and 50% relative humidity for a minimum of 7 days.
The samples were then sanded and cut using a circular saw to 5 x 5 x 1.1 cm. They were allowed to continue conditioning in the same conditions for a further minimum of 7 days.
Thickness swell was measured according to standard BS 317. The number represented in Table 1 below is the average results of 8 cut samples. Also internal bond strength IB V20 (dry) (according to standard BS 319 modified RH 50±5 %, temp 23±2°C) and IB V100 (cooked) (according to standard BS 319 modified RH 50±5 %, temp 23±2°C / EN 1087-1) was measured.

The results presented in Table 1 below show that by using polymeric MDI compositions having higher minor triisocyanate isomers content than the standard polymeric MDI composition (Ref 1) leads to boards with improved swelling performance and also improved bond strength.

**Table 1**

| Sample | Composition | Relative minor triiso (wt%) | Thickness swell (%) | IB V20 (MPa) | IB V100 (MPa) |
|---|---|---|---|---|---|
| Ref 1 | ISO 1 | 14.8 | 7.5 | 0.6281 | 0.1033 |
| 1 | ISO 2 | 22.8 | 6.1 | 0.8139 | 0.1079 |

### EXAMPLE 2

Compositions containing various polyisocyanates as identified below in Table 2 were prepared.
These compositions were used to make medium density fibreboards using pilot scale blow line (2 cm diameter and 20 m long).
Fibres were produced in situ from Western European source of mixed softwood chips (chips have been cooked at 150°C, 5 bar for 5 min.). The resinated fibres were then dried to a moisture content of 7-9 %.
Resin loading of 3 or 4 wt% on total wood composite were used.
Wood panels with dimensions 50 x 50 x 1.2 cm were produced using a two step press profile in which the panel is first pressed rapidly to 13 mm and after 75 seconds the panel is pressed to final thickness of 12 mm for a further 75 seconds. Again the press platen temperature was 220°C.
The samples were sanded, conditioned and cut to 5 x 5 x 1.2 cm dimensions.
Thickness swell was measured according to standard BS 317. The number represented in Table 2 is the average results of 8 cut samples. Also internal bond strength IB V20 (according to standard BS 319 modified RH 50±5 %, temp 23±2°C) was measured.

The results presented below in Table 2 show that by using polymeric MDI compositions having higher minor triisocyanate isomers content than the standard polymeric MDI compositions (Ref 2) leads to boards with improved swelling performance and improved bond strength (sample 2). Alternatively the same swelling performance can be obtained at lower resin loadings (sample 3).

**Table 2**

| Sample | Composition | Relative minor triiso (wt%) (wt%) | Resin loading (wt%) | Thickness swell (%) | IB V20 (MPa) |
|---|---|---|---|---|---|
| Ref 2 | ISO 3 | 16.6 | 4 | 12.5 | 0.77 |
| 2 | ISO 4 | 23.8 | 4 | 10.4 | 0.84 |
| 3 | ISO 4 | 23.8 | 3 | 12.1 | 0.85 |

## Claims

1. A process for binding lignocellulosic material comprising the steps of a) bringing lignocellulosic material into contact with a polyisocyanate composition and b) subsequently allowing said material to bind **characterised in that** the polyisocyanate composition comprises a methylene bridged polyphenyl polyisocyanate composition having a having a relative content of minor triisocyanate isomers of at least 17 wt% based on the total triisocyanate content.

2. Process according to claim 1, wherein the relative content of minor triisocyanate isomers is at least 20 wt%, preferably at 22 wt%.

3. Process according to any one of claims 1 or 2, wherein the absolute content of minor triisocyanate isomers is at least 4 wt%, preferably at least 4.5 wt%, most preferably at least 4.8 wt% based on the total polyisocyanate.

4. Process according to any one of the preceding claims wherein the methylene bridged polyphenyl polyisocyanate composition is a water-emulsifiable one.

5. Process according to any one of the preceding claims wherein the polyisocyanate composition is applied in such an amount as to give a weight ratio of polyisocyanate to lignocellulosic material in the range 0.1:99.9 to 20:80, preferably in the range 0.5:99.5 to 10:90 and most preferably in the range 3:97 to 8:92.

6. Process according to any one of the preceding claims wherein step b) involves pressing the lignocellulosic material, preferably at 120°C to 300°C and 2 to 6 MPa specific pressure.

7. A binder for lignocellulosic material comprising a methylene bridged polyphenyl polyisocyanate composition having a having a relative content of minor triisocyanate isomers of at least 17 wt% based on the total triisocyanate content.

8. A polyisocyanate composition used for binding lignocellulosic materials comprising a methylene bridged polyphenyl polyisocyanate composition having a having a relative content of minor triisocyanate isomers of at least 17 wt% based on the total triisocyanate content.
